(19) **Europäisches Patentamt**
**European Patent Office**
**Office européen des brevets**

(11) **EP 2 342 586 B1**

(12) **EUROPEAN PATENT SPECIFICATION**

(45) Date of publication and mention
of the grant of the patent:
**06.11.2013 Bulletin 2013/45**

(51) Int Cl.:
***G01T 1/164*** *(2006.01)*

(21) Application number: **09787821.9**

(22) Date of filing: **03.08.2009**

(86) International application number:
**PCT/IT2009/000355**

(87) International publication number:
**WO 2010/018606 (18.02.2010 Gazette 2010/07)**

(54) **DETECTION OF SMALL BREAST TUMOURS BY RADIONUCLIDE IMAGING**

NACHWEIS KLEINER MAMMATUMOREN MITTELS RADIONUKLIDBILDGEBUNG

DETECTION DE PETITES TUMEURS DU SEIN PAR IMAGERIE ISOTOPIQUE

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **11.08.2008 IT RM20080451**

(43) Date of publication of application:
**13.07.2011 Bulletin 2011/28**

(73) Proprietor: **Istituto Superiore di Sanità
00161 Roma (IT)**

(72) Inventors:
• **GARIBALDI, Franco
I-00198 Roma (IT)**

• **CISBANI, Evaristo
I-00159 Roma (IT)**
• **GRICIA, Massimo
I-00159 Roma (IT)**

(74) Representative: **Perronace, Andrea
BARZANÒ & ZANARDO
26, Via Piemonte
00187 Roma (IT)**

(56) References cited:
**US-A1- 2001 001 107    US-A1- 2007 221 853
US-B1- 6 583 420**

**Description**

The present invention concerns a device for the detection of small tumors in the diagnosis of breast cancer by means of molecular imaging with radionuclides.

More precisely, the present invention concerns a device that uses two coupled detectors and, for the first time, with different dimensions and collimation geometries: a detector with standard dimensions having parallel-hole collimator and, in front of this, a detector with smaller dimensions having pinhole collimator, with reduced field of view (FOV) so as to allow the use of the spot compression technique. This device is suitable to detect lesions and to establish their nature.

The breast cancer is the most widespread tumour in the woman. Its successful treatment is connected to the early diagnosis, therefore to the detection of small tumors ($< 10$ mm, if possible $\leq 5$ mm) that makes possible therapeutic operations presumably without metastasis. The most utilised diagnosis technique is the X-rays mammography, very much sensible but not specific, that does not provide therefore, actually, information on the nature of the lesions [1]. The mammography is moreover less reliable in case of dense mammary tissue, mammas with prosthesis or surgically treated mammas; and it is known that women with dense breast have a much more higher risk of contracting the cancer with respect to the other ones.

In the usual clinical procedure, in those cases that are uncertain on the basis of the mammographic screening, one executes the biopsy. Such a technique is known to be invasive, anxiety-inducing and expensive. A great number of biopsies prove not necessary (false positives) [1,2,3]. One can also have a certain number of false negatives.

In case of doubts on the nature of lesions, one has to utilise functional techniques, in particular those based on the use of the radionuclides.

The up-to-now most used functional technique is scintimammography in the prone position by means of MIBI marked with 99mTc with the standard Anger camera that is comprised in the equipments of nuclear medicine divisions [3,4]. This has proved an ideal complement to the X-rays mammography because it is able to visualise the metabolic activity of cancerous lesions [4], but it does not allow the detection of $T1_a$ and $T1_b$ tumors ($< 10$ mm) [3,5].

The limitation is due to the geometry of the detector (the considerable overall dimensions do not allow the optimal adaptation to the organ) and to its characteristics. The spatial resolution is indeed limited by the intrinsic spatial resolution and the distance tumour-collimator. One has utilised also techniques of Single Photon Emission Computed Tomography (SPECT), with different collimators and modalities, without important improvements.

It has been needed therefore to construct high resolution and high efficiency dedicated detectors [6,7], that better adapt to the organ and therefore allow to execute in-compression measurements, with the reduction of the distance tumour-detector and consequent decrease of the background noise and increase of the signal. The diagnostic ability has improved. One has performed clinical tests with single detectors and more recently with two coupled detectors [7,9]. The number of clinical tests performed up to now is however not sufficient and therefore the technique is not currently used.

Moreover, to detect very small tumors in a reliable way, it is necessary and possible, as it will be demonstrated, to further improve the detectors performances.

It is to be stressed here the fact that the poor sensitivity and specificity of the mammography in the women with prosthesis or with dense breasts (and the relevant much higher risk of contracting cancer) makes it clear the importance of improving devices and use modalities of techniques that are complementary and/or alternative those today in use.

It is object of the present invention that of providing a device allowing to increase in a conclusive way the diagnostic abilities for the tumors $T1_a$ and $T1_b$, by improving the intrinsic characteristics of the detectors, the collimation techniques and the operation modalities, at least partially solving the problems of the prior art and overcoming its drawbacks.

US 6 583 420 discloses a radionuclide scintimammography device comprising one detector and two compression plates. By applying the detector onto the compression plates, gamma rays can be detected; moreover, the compression plates are adapted to realise a spot compression in order to reduce the distance between the detector and a potential internal lesion.

It is subject-mater of the present invention a device for detecting small tumours or lesions in the diagnosis of breast cancer by means of molecular imaging using radionuclides, comprising a first and second detector, between which the mamma is to be positioned, characterised in that:

- said second detector has the same dimensions of the mammographic film and said first detector has dimensions that are smaller than those of the mammographic film and such to be suitable to realise a spot compression and therefore reduce the distance lesion-detector;
- said second detector is fixedly mounted on a support that extends in a direction $z$;
- said first detector is mounted:

  o on a mobile support along said direction $z$, for the compression of the mama,
  o in a rotatable way on said mobile support so as to be suitable to form an angle $\alpha$ between 0 and 90 with the direction $z$, for minimizing the distance lesion-detector in the cases of tumours close to thorax, and

o in a movable way in the *xy* plane on said mobile support for the optimal positioning with respect to the mamma.

Preferably according to the invention, said first detector uses a collimator of the pinhole type.
Preferably according to the invention, said second detector uses a collimator of the parallel holes type.
Preferably according to the invention, said first detector uses a NaI(Tl) pixeled scintillator.
Preferably according to the invention, said first detector uses a LaBr$_3$ (Ce) scintillator.
Preferably according to the invention, the ratio between the pixel dimensions of the scintillators are between 1 and 1.4 mm for the first and second scintillator.
Preferably according to the invention, said second detector uses a LaBr$_3$ (Ce) scintillator.
Preferably according to the invention, said second detector uses a NaI(Tl) pixeled scintillator.
Preferably according to the invention, the dimensions of said first detector is of 45-55 x 45-55 mm$^2$ in order to allow the spot compression.
Preferably according to the invention, the FOV is varied using plastic spacers with this window, in order to allow the optimisation of the FOV trade-off efficiency-spatial resolution for the optimisation of detectability of small lesions.
The invention will be now described by way of illustration but not by way of limitation, with particular reference to the figures of the enclosed drawings, wherein:

- figure 1 shows a schematic plant view of the detection system and the moving system xyz;
- figure 2 shows three lateral views of the detection system: (a) the in-compression large detector (with parallel-hole collimator); (b) the small detector with localised compression (spot compression); (c) the in-compression small detector: advantages in the detection of tumors close to thorax are evident;
- figure 3 shows a comparison diagram between the spatial resolutions of two detectors having parallel-hole and pinhole collimators as a function of the source-detector distance;
- figure 4 shows the progression of the efficiency and the FOV of detectors with parallel-hole and pinhole as a function of source-detector distance;
- figure 5 shows the advantages of the use of spacers in the pinhole detector;
- figure 6 shows the progression of efficiency and FOV for the detectors with parallel-hole and pinhole collimators as a function of source-detector distance, for the different dimensions of the spacers (see figure 5) (c2,c4,c5) and therefore different values of the FOV (FOV-2, FOV-4, FOV-6) and the efficiency;
- figure 7 shows the progression of the efficiency of the dual detection system in the case of two detectors with parallel-hole collimators (par-par) or a detector with parallel-hole collimator and the other one with pinhole collimator;
- figure 8 shows a comparison diagram of the SNR with single detector having parallel-hole collimator (measured values) with what obtainable (values extrapolated by the efficiencies calculated in the figures 4, 5, 6) using two parallel-hole detectors and dual detector (detectors with parallel-hole collimator coupled with a spot compression small detector having pinhole collimator).

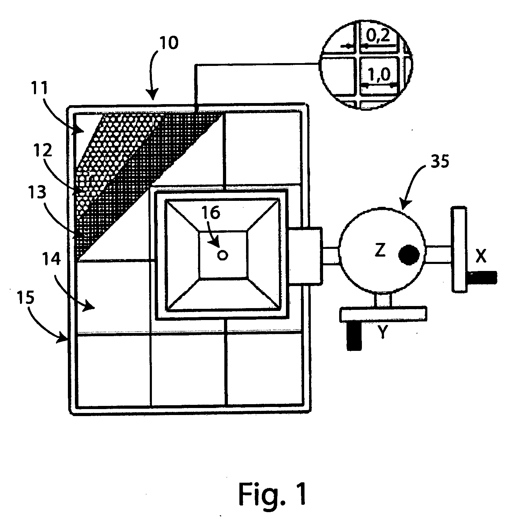

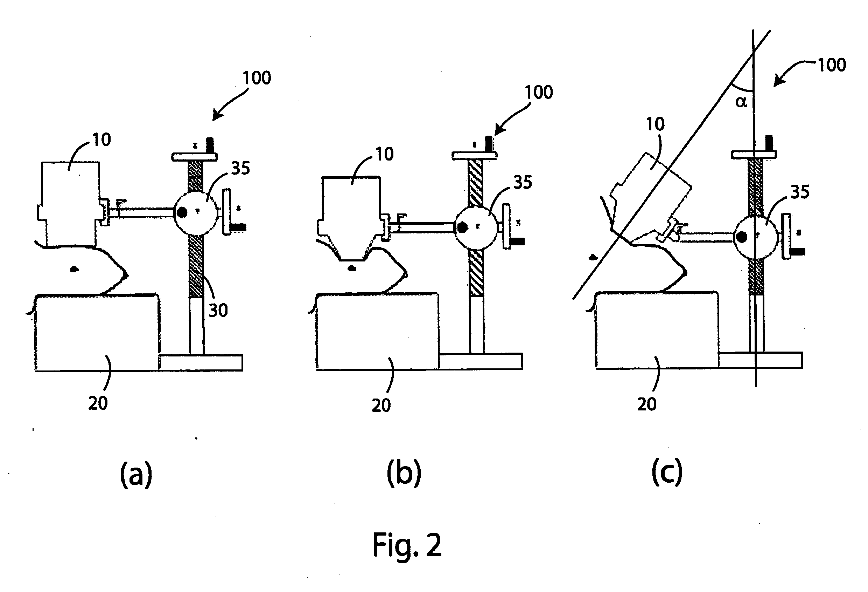

In the described embodiment, making reference to figure 1 and 2, the device 100 according to the invention comprises two detectors 10,20 of different dimensions, each constituted by a collimator, scintillator, photodetector, electronics, acquisition system, data analysis and elaboration (not shown).
The two detectors 10,20 are mounted on a trestle support 30 which allows the "standard" moving (along z) for the compression, the moving along xy for the optimal positioning of the smallest detector (figure 1 and 2) and the rotation of the same around an axis perpendicular to z.
To a detector having standard dimensions (150 x 200 mm$^2$ as for the X-rays mammography) with parallel hole collimator, a detector having smaller dimensions (50 x 50 mm$^2$), and with collimator of the pinhole type, is faced, which has a reduced FOV so as to allow the use of the so-called spot compression technique.
The proposed modality, and the use of pinhole collimator for the smaller detector, allow to maximise the detection efficiency and spatial resolution, and therefore the signal-to-noise ratio (SNR), which are critical parameters in the detection of very small tumors, what constitutes decisive element for the diagnosis and the therapeutical planning.
Before describing in greater detail the functioning of the device according to the invention, one gives some remarks on important aspects concerning the same device.

Detectability of tumors

The fundamental parameters for the detectability of a tumour are the spatial resolution, contrast and the SNR, as defined by the following relationships.
The spatial resolution $\sigma_x$ (along the arbitrary direction *x*) of the detector is defined by the standard deviation of the detector response function (DRF) relevant to the function of spatial distribution or "Point Spread Function" of the photons produced by the interaction of gamma rays (coming from a point-like source) with the scintillator.
Image contrasts (IC) is given by:

$$IC = (Max-BKG)/Max$$

wherein Max = maximum of the counts on signal pixels, while BKG is the average count in a background ROI (Region of Interest).

Finally the SNR is defined by:

$$SNR = \sqrt{\frac{S - BKG}{S}}$$

Wherein S are the counts of the ROI defined around the image of the signal.

In the case of the device according to the invention, the FOV of the larger detector must have dimensions at least equal to the organ under examination for the larger detector and sufficiently small (see below) for the smaller dimensions detector.

Given the limited dimensions of the human organ to be analysed, the energy resolution plays a role that is important but less considerable [7,17].

The quantities introduced are closely related to each other. They depend on the intrinsic characteristics of the detector, the collimation system and measurement modality.

The SNR and the contrast depend both on the spatial resolution (and therefore on the pixel dimensions [10, 11, 12] ) and the efficiency, which depends in turn on the source (tumour)- collimator distance and the collimation system.

Geometry of the detection system

It is possible, with a suitable choice of the scintillator and its geometry, the photo-detector, and reading electronics and the data acquisition system electronics, to build up a detector with highest intrinsic spatial resolution (around 1 mm) and high efficiency.

In order to maximise the detectability of small tumors, it is proposed the use of two detectors, of different dimensions (for example 150 x 200 mm$^2$ - standard dimensions of the mammographic film - and 50 x 50 mm$^2$), in compression, in such a way to reduce the background and the distance of source-detector, which are coupled, for example in the two projections normally used in high-resolution mammo-scintigraphy (cranium-caudal and oblique middle-lateral).

The information coming from the two detectors must be co-saved and combined in a suitable way so as to maximise the increase of the spatial resolution and system efficiency with respect to the single detector [9].

In the case of the device according to the invention, one of the detectors has the dimensions of the mammographic film, the other one has reduced dimensions so as to make it possible a higher compression of the mamma and therefore to bring the tumour still closer to the detector (spot compression).

One therefore obtains a clear improvement of the spatial resolution and the sensitivity (efficiency) and therefore of the SNR and the contrast [9].

Making reference to figure 1, the smallest receiver in the device according to the invention comprises an aluminium sheet 11 of 0.5 mm, a Pb hexagonal-holes collimator, a 150 x 200 mm pixelled crystal 13, photomultipliers 14, and an external tungsten casing 15, a compression detector 16 with pinhole, a xyz moving system 35.

The use of a pinhole collimator for the smallest detectors further allows to improve both the spatial resolution (with respect to the parallel hole one) and, particularly, the sensitivity (efficiency), which is a dominant parameter for the detectability.

It is thus improved the detectability small tumors, in a decisive measure.

The use of a pinhole collimator entails the reduction of the FOV, but this is a positive factor just because it allows a larger compression (and therefore a larger source-lesion/tumour distance).

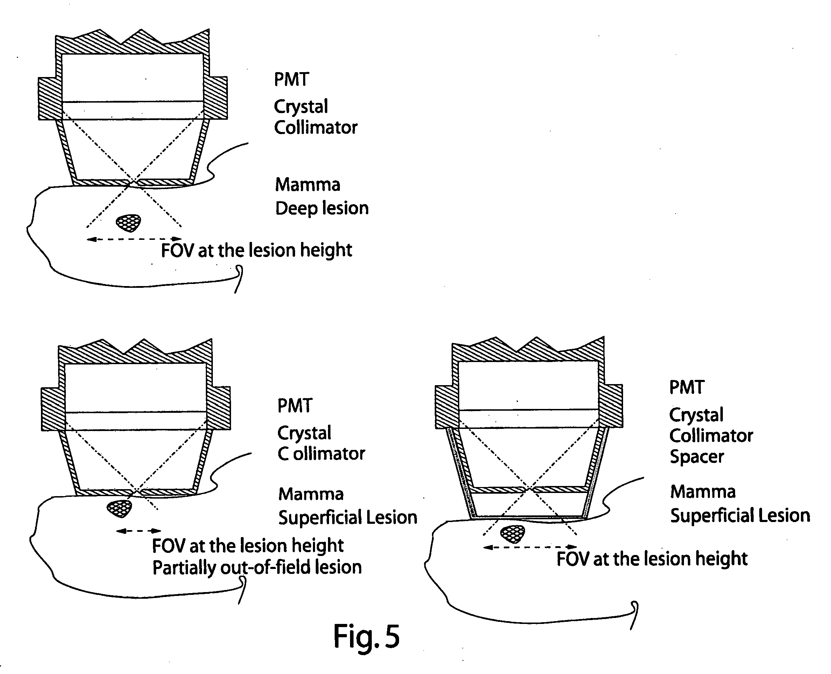

The FOV, variable as a function of the source-detector distance, must be in any case sufficient for the detection of small dimensions tumors (around 5 mm). In particular, making reference also to figure 5, the use of spacers of suitable dimensions allows, if necessary, to change the FOV (figures 1, 2, 5, 6) as a function of the characteristics of the suspected lesion (for example position and multi-focality). For lesions that are not of multi-focal nature, a FOV of 25 x 25 mm$^2$ will be sufficient, with evident advantages in terms of sensitivity.

The spacers can be realised in thin plastic. It is possible to vary the FOV by modulating the source-collimator distance (detector) (trade-off of FOV, efficiency, spatial resolution). One focuses the tumors detector in the position A. If the morphological exams (mammography, echography, MRI) show that the detector is in a different position (for example B), in order to hold constant the FOV, one uses just the thin plastic spacers, different as a function of the position. One can thus optimise the trade-off FOV-efficiency-resolution.

The particular geometry of the proposed system, i.e. with a movable and adjustable small detector (figure 1 and 2), allows to get close to the lesion more than it is possible by using normal dimensions detectors, with clear advantages in terms of the detectability.

The set of the techniques of morphological imaging (mammography, echography, if possible MRI) allows indeed to localise with high precision the suspected lesions and therefore to position in the correct way the small detector in the zone of the lesion with a FOV that is function of the particular case under examination (FOV variable from around 15 millimetres to around 45 mm).

Collimation systems

The emission from the source is isotropic, therefore, in order to construct an image of the same, it is necessary to collimate the emitted gamma rays. This entails a remarkable decreasing of the detection efficiency.

The proposed collimation system is different for the two detectors. It deals with a high efficiency parallel-hole collimator for the large detector 20 and a pinhole collimator for the small detector 10.

As seen above, the pinhole collimator plays a crucial role in the proposed system.

Parallel-hole collimator

The efficiency and the spatial resolution of a parallel-hole collimator are calculated by using the for-mulae here quoted:

$$R'' = \sqrt{R_i^2 + R_c^2}$$

Wherein $R_i$ is the intrinsic resolution of the scintillator and

$$R_c = d\left(\frac{L+b}{L}\right)$$

That of the collimator, with $L$ the collimator thickness, $d$ the hole diameter and $b$ the distance between source and collimator. $R_i$ is equal to the pitch of the scintillator pixel.

The parallel-hole collimator efficiency is given in a first approximation by:

$$\varepsilon'' \approx K\left(\frac{d}{L}\right)^2\left(\frac{d}{d+t}\right)^2$$

$K$ being a constant which depends on the type of hole ($K$ = 0,26 for hexagonal holes) and $t$ the thickness of the separator between holes.

By combining the preceding formulae one can write approximately:

On the basis of analytic calculations, Monte Carlo simulations and measurements on phantoms, the following geometry has been chosen, which is a reasonable compromise in terms of sensitivity and spatial resolution (therefore of SNR): hexagonal holes $d$ = 1.9 millimetres, $L$ = 22 millimetres.

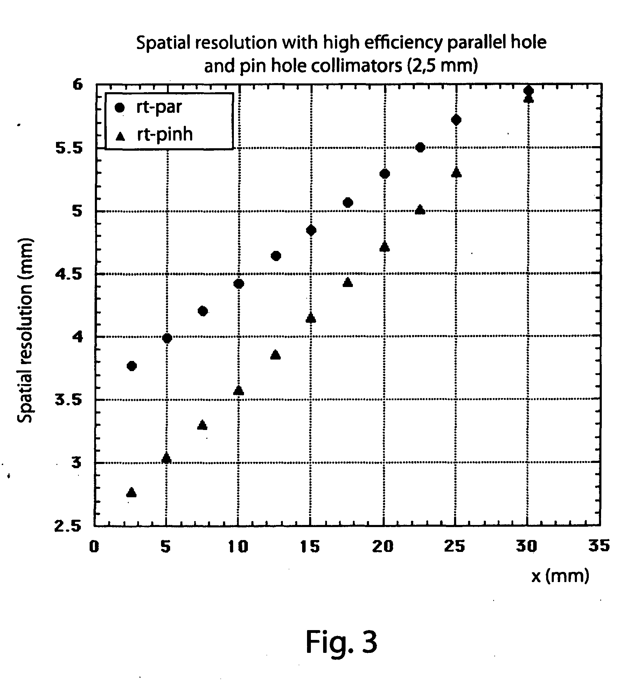

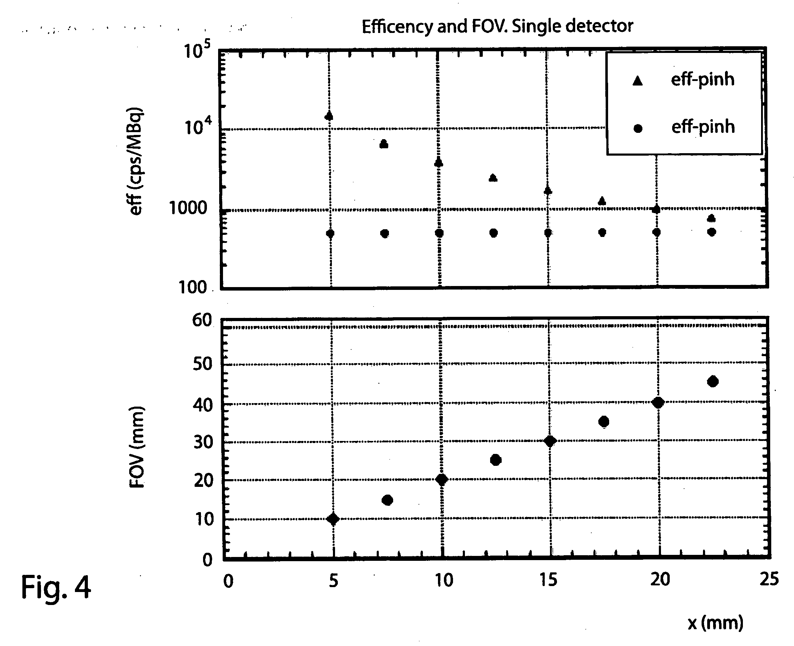

Sensitivity and spatial resolution of the detector, as a function of different intrinsic resolutions of the same and different source-detector distances, are reproduced in the figures 3,4,6 and compared with those of the (pinhole) small detector.

Pinhole collimator

The efficiency and spatial resolution $R°$ of a pinhole collimator are calculated by the following formulae:

$$R° = \frac{x}{f}\sqrt{R_i^2 + \left(\frac{f+x}{x}\right)d_e^2}$$

Wherein $x$ is the distance between source and pinhole, $f$ is the distance between the pinhole and the intrinsic detector (scintillator) and $d_e$ is the pinhole effective aperture, given by:

$$d_e = \sqrt{d\left(d + \frac{2}{\mu}\tan(\alpha/2)\right)}$$

Wherein $d$ is the pinhole aperture, $\mu$ is the attenuation coefficient of the pinhole material and $\alpha$ is the pinhole aperture angle.

The efficiency $\varepsilon$ is given by:

$$\varepsilon = \frac{d_e^2\cos^3\vartheta}{16x^2}$$

Wherein $\vartheta$ is the angle of the trajectory between the source and the pinhole with the pinhole axis (zero for the source on the axis) and $d_e$ and $x$ are defined as above.

The use of the pinhole collimator allows to modulate, particularly if the source-hole distance is (relatively) small, in a profitable way with respect to the parallel hole, the spatial resolution and the sensitivity, to the detriment of the FOV.

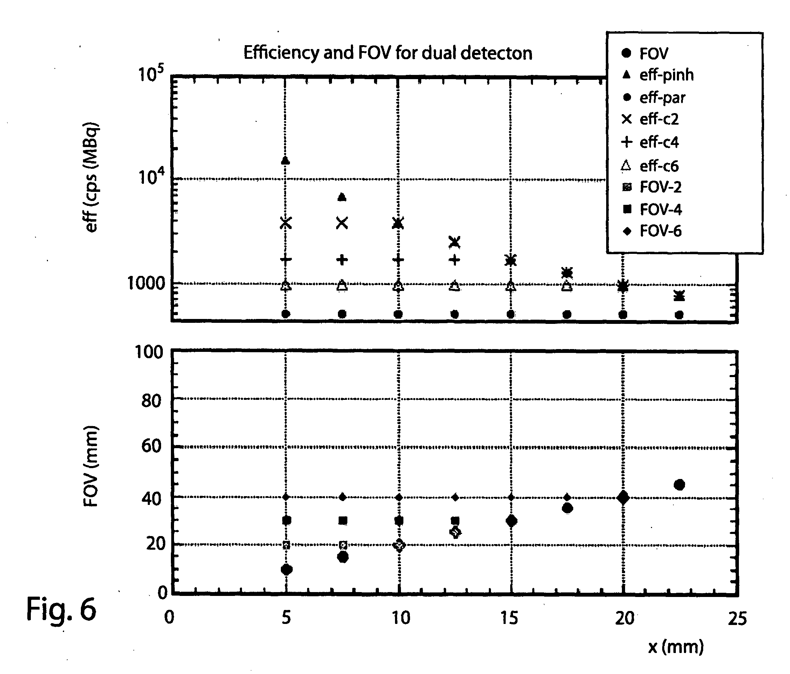

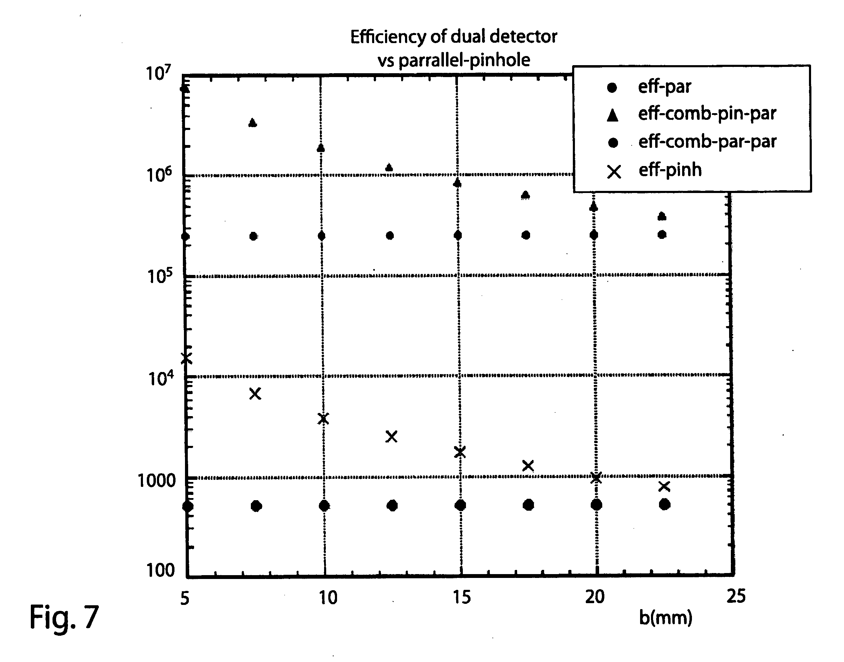

Figures 6 and 7 show the comparison between deficiencies of the single detectors with parallel-hole collimator and pinhole, and those of the dual detectors (parallel hole/parallel hole and pinhole/parallel hole). The advantages of the proposed detector are evident both for the single detectors and for the dual detectors.

The considered pinhole collimator has a hole of 2 mm and magnification of 2 in the focus (12.5 mm distance from the surface). One can consider a fine tuning of the two parameters.

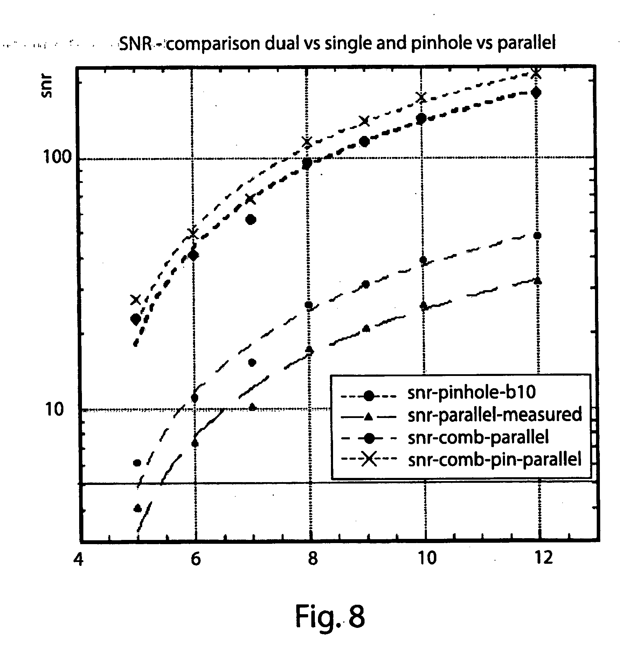

Figure 8 shows the comparison in terms of SNR (therefore the detectability of the tumour) between the different configurations (single parallel hole and pinhole detectors and parallel hole/parallel hole and pinhole/parallel hole dual detectors).

Scintillator

The choice of the scintillator depends on the following characteristics: detection efficiency of the incident gamma rays (it influences the global efficiency of the system), light yield (it influences the energetic and spatial resolutions, and therefore the contrast and the SNR), photo-fraction (it influences the efficiency and the quality of the image), the dimensions (it influences, the width of the light distribution generated and therefore the intrinsic spatial resolution).

The NaI(Tl), for its characteristics (efficiency, light yield, photo-fraction, etc.) is the most used scintillator in the Anger camera and in different high-resolution detectors, that are dedicated to the mamma imaging [20]. Other solutions are possible also as a function of the technological evolutions of the technical domain.

Continuous scintillator

The scintillator can be continuous or pixelled. For the larger detector the use of continuous scintillators is, in practice, today not proposable because it is not at disposal with the needed characteristics.

The use of a continuous NaI(Tl), that has a thickness sufficiently thin to allow intrinsic spatial resolutions of the order of those needed (around 1 mm) would damage the efficiency.

It appeared on the market in relatively recent times a new scintillator, the LaBr3(Ce) [22] that has a most remarkable light yield and characteristics that render it usable, in line of principle, for the highly-resolution mammo-scintigraphy. It is however impossible for the time being to construct plates having the dimensions needed for the larger detector.

The choice will be reconsidered at the time of possible overcoming of the nowadays technological limitations.

The LaBr3(Ce) can be instead constructed in the dimensions needed for the small detector (scintillator of dimensions 50 x 50 x for $mm^3$ are available). The intrinsic spatial resolution is slightly larger than 1 mm.

Pixelled scintillator

The characteristics of the pixel scintillator (NaI(Tl)), but other solutions are possible as a function of the technological evolutions in the field, are dictated by the requests on the intrinsic spatial resolution. Small pixels allow to obtain better spatial resolutions (and SNR). It is here to be stressed that this is true provided that the pixels are well identified and their content in terms of counts is statistically significant. The step of the sampling of the light emitted as a consequence of the interaction of the gamma rays with the scintillator and electronics plays a crucial role in this.

In the choice of the pixel dimensions is however limited, on one side by the technology, and on the other side by practical and cost considerations.

It is nowadays impossible to construct in a reliable way NaI(Tl) matrixes having pixel < 1.0 mm + 0.2 mm (dead area). The minimal pitch is therefore of 1.2 mm.

It is here to be stressed that the choice of the LaBr3 for the small detector allows to obtain for it an energy resolution definitely better than that it is possible using a pixelled NaI(Tl).

As we will see later on, this can be important in case of tumors close to the thorax (the Compton background is larger and therefore the energy resolution is particularly important).

Photo-detectors

One has considered different photo-detectors, in particular photo-tubes that are sensible to the position (Position Sensitive Photomultiplier Tube, PSPMT) Hamamatsu H9500 (anodo 3 x 3 $mm^2$) and H8500 (anodo 6 x 6 $mm^2$). There are photo-tubes having anodes of different dimensions but with geometric characteristics such that one has to advise against their use because of the increasing of the dead area.

Choice criteria of the sampling step

The system of photo-detectors must be able to sample in a suitable way the light emitted as a consequence of the interaction of the gamma rays with the scintillator.

It is necessary, in other words, to use a suitable sampling step. One has performed simulations of the involved physical processes, by means of the code GEANT4, for the choice of the sampling step. The chosen merit factor is the distribution of the standard deviation of the reconstructed position of the interaction of the gamma rays in the scintillator with respect to the "true" positions.

A ratio around 2 between anodic pixel dimensions and scintillator pixel dimensions seems to be the reasonable compromise.

It is evident that the best choice today is: NaI(Tl) 1.2 mm pitch coupled with a H9500 (3x3 $mm^2$). One has to bear in mind however that this choice entails an increase in the complexity of the system because of the large number of the involved channels.

A possible alternative is the NaI(Tl) 1.5 mm pitch coupled with a H8500 (6 x 6 $mm^2$).

For the small detectors analogous considerations hold: La Br3 (Ce) coupled to phototubes H9500 [18,21].

Alternative solutions

There are other potentially usable photo-detectors, such as the MicroChannel Plate (MCP) of the Burle [23] and the Silicon PhotoMultipliers (SiPM) (24).

The MCP has the same dimensions of H9500 and H8500, and interesting characteristics such as the possibility to construct, in a relatively simple way, in line of principle, anodic pixels of the wished dimensions, the good gain uniformity among various channels and insensibility to magnetic fields.

The SiPM have very interesting characteristics for this type of applications. It deals with solid-state detectors of small dimensions, not much bulky, and with high quantum efficiency.

They are however, for the time being, available only for small matrixes, still not technologically "mature" for the detectors that must be used in clinic.

Their possible utilisation will be reconsidered and proposed at the moment of the overcoming of the nowadays technological difficulties.

Electronics for the acquisition of signals

One uses the system with individual reading of all the channels. The here proposed electronics (1024 channels with reading speed up to 20 kHz) has a certain number of advantages with respect to the generally used resistive chains:

- larger sensitivity, connected to the fact that the trigger can be set on the single channel and in such a way to take into account the disuniformities of these channels. This, besides, can allow a better selectivity of the good events;
- negligible interference among channels and therefore, in general, better quality of the acquired signal;
- better flexibility, that allows, besides, to take into account the disuniformities of the detectors and also of possible "hot channels", in a transparent and rapid way (without intervening on the hardware);
- remarkable possibilities of processing of the acquired data and therefore better ability of compensating disuniformities and spatial distortions without compromising the other image quality parameters.
- better identification of the pixels, thanks to all the advantages just listed.

System of data elaboration

The digitised data are transmitted to the computer and elaborated by means of algorithms, subject to calibration of the system. But vibration is performed by measurements with ad hoc configuration and source processed by the appropriately developed programmes.

It consists in:

- equalisation of the gains of the single anodes: one acquires, by irradiating the detector with a uniform extended source (flood) and one analyses the energy spectra of each anode. One estimates the gain correction by measuring the slope of the logarithmic tail of each spectrum, i.e. by the average besides the pedestal;

- geometrical distortions correction: one acquires by irradiating the detector with the uniform source in the case of a pixelled crystal (i.e. a series of acquisitions with point-like source placed on a regular grid for a continuous crystal) with and without collimator. The obtained image of the centroids (pixels of the crystals or image grid), more or less distorted, is rectified by means of the semi-automatic procedure on regular grid. The correspondence between homologous points is exploited to construct a Delaunay triangulation and subsequently one proceeds to linear interpolation, or of higher order interpolation, depending on the distortion size;
- determining energetic acceptance window as a function of the position: still on uniform source image, on the same grid of the preceding step, one determines energy spectra (as a function of the grid point) and therefore, on each one the photoelectric peak and the consequent energetic cuts are selected (at a suitable distance from the centre of the photopeak) ;
- equalising the crystal response: from uniform source, once applied the preceding corrections, one determines, on regular grid, the average values of the counts and renormalizes them to the average count, providing in such a way the equalisation coefficients.

Once one has determined the calibration parameters, one proceeds to the data processing through the following steps:

- pedestals subtraction;
- digital data conversion into energetic units;
- gain corrections (coefficients as determined in step 1 of the calibration);
- centroids calculation (as obtained by the weighted average of the charge deposed on the single anodes, in the simplest version);
- energetic acceptance windows according to what has been obtained in the calibration;
- centroids projections onto the regular grid by means of interpolation;
- equalisation correction by the application of the coefficients as derived in the calibration.

Operation modalities

The advantages of the system according to the invention are evident. Also the here described operation modalities can have an important role in the detection of small tumours.

The large detector provides the global image of the mamma by individuating also the possible multi-focal nature of the lesion. It gives a different contribution as a function of the distance from the tumour in the two operation positions (for example cranium-caudal and oblique medium-lateral or two cranium-caudals projections with detectors in swapped positions), which can be determinant.

Subsequent examinations on the other focal points will allow a more complete diagnosis.

One proposes the following operation modalities:

a. One individuates the position of the suspected lesion by means of mammography, echography and, where possible, MRI;

b. One performs of measurements in cranium-caudal projection: one positions the large detector in that part which is closer to the tumour and the smaller one in the opposite part. The image of the large detector, combined the with that of the small detector 9 will provide information on the lesion, and, if possible, on the multi-focal nature of the same;

c. One reverses the position of the detectors. In such a case, the suspected lesion will be close to the small detector. The signal, once suitably combined 9 with that of the other detector, will make the SNR significantly increase (what is decisive in case of very small lesions), and the same hols also for the contrast and therefore the detectability of the tumour.

d. One performs the measurement in oblique lateral medium position.

A further advantage of the system and the operation modalities according to the invention is given, especially in case of tumours that are close to the thorax, as mentioned above, by the possibility of rotating the small detector (figures 1,2). This allows to position the same closer to tumours which are close to the thorax, what is a very difficult thing using large detectors. In such cases, it is moreover well known that the tumour detection is more difficult owing to the higher background that is emitted by the thorax. It is also for this reason that one proposes the use of LaBr3(Ce) for the small detector as preferred solution. As noted above, indeed the LaBr3 allows to obtain a better energetic resolution and therefore it is possible to better subtract the Compton background, with evident advantages for the image quality. The particular geometry is in any case advantageous with respect to the other systems, even using the NaI(Tl).

It is here proposed with the present invention a system for measurements in molecular imaging using radionuclides, to be used as ancillary technique with respect to those used in senology or screening for risk women (see O'Connor et al. [25]).

Such a system has the following advantages with respect to all the today commercialised systems or the systems described in the technical-scientific literature:

- the intrinsic characteristics of the detectors are today the most advanced ones on the market for detectors that are constituted by scintillator plus photodetector, for:

  - scintillator pixel dimensions, which are smaller than the today commercialised systems (advantages: higher pixels amount in the image, better intrinsic spatial resolution, larger SNR);
  - electronics for the acquisition of all the channels; it allows to better identify the pixels especially at the detector edges and in the dead zones;

- it uses at the same time high resolution detectors with dual technique (two detectors);
- it uses detectors having different dimensions for the spot compression (advantages: larger compression, decrease of the source-detector distance, increase of the signal and spatial resolution, decrease of the background, consequent increase of the SNR and contrast and therefore detectability of the small tumours (that is an extremely important factor for the choice of the therapy).
- possibility of rotation of the small detector (it has better energetic resolution (one uses LaBr3)); one can therefore position it closer to the suspected lesion and in positions close to the thorax and therefore, based on what has been above explained (improvement of the spatial resolution, efficiency and SNR), one has a consequent improvement of the detectability of the tumour;
- cost smaller with respect to the other dual systems (in the case of the present invention one of the detectors has smaller dimensions and requires a corresponding lower production cost).

The preferred embodiments of this invention have been hereinbefore described and some variations of this invention have been suggested, but it should be understood that modifications and changes can be made by those skilled in the art without so departing from the protection scope as defined by the annexed claims.

Bibliography

1. D.B. Kopans, AJR 158 (1992) 521, Bird RE et al. Analysis of cancers missed at screening mammography", Radiology 1992, 184:613-617

2. D. Cyrlak, Radiology 168, 1988, 661

3. F. Scopinaro et al. NIM A 497, 2003, 14-20

4. I. Khalkhali, et al. J. Am Coll. Surg. 178, 1994, 491

5. G. De Vincentis et al. NIM A 497, 2003, 46-50

6. D. Kieper et. Al, NIMA 497, 2003, 168-173

7. C.B. Hruska et al, Physica Medica 21, Suppl 1, 2006, 72

8. R. Pani et al. NIM A 392, 1997, 295

9. P.G. Judy et al, IEEE-NSS 2007 Conference records (M-20-1), 4040-4043

10. G.J. Gruber et al., IEEE TNS NS 46, 1999, 2119 - 2123

11. F. Garibaldi et al. NIM A 569, 2006, 286 - 290

12. M.N. Cinti et al IEEE TNS 50 (5), 2003, 53-59

13. E. Cisbani et al. NIM A 571, 2007, 169-172

14. F. Cusanno et al. NIM A 569, 2006, 193-196, and references quoted therein

15. Saint-Gobain Crystals, personal communication (March 2007)

16. R. Pani et al. NIM A 567, 2006, 294-297

17. F. Garibaldi et al. Nucl. Instr. Meth A 471, 2001, 222-228

18. M.L. Magliozzi et al. Proceedings of 9th ICATPP Conference, Como 2005)

19. O' Connor, personal communication on a paper to be published on Am. J. Roentgentology

20. K. Madsen, THE JOURNAL OF NUCLEAR MEDICINE • Vol. 48 • No. 4 • April 2007

21. M. L. Magliozzi et al, GEANT4 Code for Optimization of Molecular Imaging Detector, ISTISAN Reports 06/14 p. 15-19.

22. Saint-Gobain Cristals, BrilLanCe Scintillators performance summary, Scintillation Products Technical Note,Dec 2007,

23. See for example on http://www.burle.com/applicatnotephotom.htm

24. See for example on http://www.sensl.com/Products/ and http://www.vertilon.com/spm.html

25. MK. O'Connor et al., Proc. Of SPIE Vol. 6319, 6319 D-1 6319 D-15, (2006).

## Claims

1. Device for detecting small tumours or lesions in the diagnosis of breast cancer by means of molecular imaging using radionuclides, comprising a first and second detector, between which the mamma is to be positioned, **characterised in that**:

   - said second detector (20) has the same dimensions of the mammographical film and said first detector (10) has dimensions that are smaller than those of the mammographic film and such to be suitable to realise a spot compression and therefore reduce the distance lesion-detector;
   - said second detector (20) is fixedly mounted on a support (30) that extends in a direction *z*;
   - said first detector (10) is mounted:

     o on a mobile support (30) along said direction *z* (30), for the compression of the mama,

     o in a rotatable way on said mobile support so as to be suitable to form an angle $\alpha$ between 0 and 90 with the direction *z*, for minimizing the distance lesion-detector in the cases of tumours close to thorax, and

     o in a movable way in the *xy* plane on said mobile support for the optimal positioning with respect to the mamma.

2. Device according to claim 1, **characterised in that** said first detector (10) uses a collimator of the pinhole type.

3. Device according to claim 1 or 2, **characterised in that** said second detector (20) uses a collimator of the parallel holes type.

4. Device according to any claim 1 to 3, **characterised in that** said first detector (10) uses a NaI(Tl) pixelled scintillator.

5. Device according to any claim 1 to 3, **characterised in that** said first detector (10) uses a $LaBr_3$ (Ce) scintillator.

6. Device according to any claim 1 to 5, **characterised in that** the ratio between the pixel dimensions of the scintillators are between 1 and 1.4 mm for the first and second scintillator.

7. Device according to any claim 1 to 6, **characterised in that** said second detector (20) uses a $LaBr_3$ (Ce) scintillator.

8. Device according to any claim 1 to 6, **characterised in that** said second detector (20) uses a NaI(Tl) pixelled scintillator.

9. Device according to any claim 1 to 8, **characterised in that** the dimensions of said first detector (10) is of 45-55 x 45-55 $mm^2$ in order to allow the spot compression.

10. Device according to any claim 1 to 9, **characterised in that** the FOV is varied using plastic spacers with this window, in order to allow the optimisation of the FOV trade-off efficiency-spatial resolution for the optimisation of detectability of small lesions.

## Patentansprüche

1. Vorrichtung zum Nachweis kleiner Tumore oder Läsionen bei der Diagnose von Brustkrebs mittels molekularer Bildgebung unter Verwendung von Radionukliden, umfassend einen ersten und zweiten Detektor, zwischen denen die Brust zu positionieren ist, **dadurch gekennzeichnet, dass**:

   - der zweite Detektor (20) dieselben Dimensionen des mammographischen Films aufweist und der erste Detektor (10) Dimensionen aufweist, die kleiner als jene des mammographischen Films sind, und so geeignet ist, eine Spot-Kompression zu realisieren und daher den Abstand Läsion-Detektor zu verringern;
   - der zweite Detektor (20) fest auf einem Träger (30) angebracht ist, der sich in einer Richtung *z* erstreckt;

- der erste Detektor (10) angebracht ist:

• auf einem mobilen Träger (30) entlang der Richtung *z* (30), zur Kompression der Brust,
• in einer rotierbaren Weise auf dem beweglichen Träger, um so geeignet zu sein, einen Winkel α zwischen 0 und 90 mit der Richtung *z* zu bilden, zur Minimierung des Abstandes Läsion-Detektor in den Fällen von Tumoren nahe zum Thorax, und
• in einer beweglichen Weise in der *xy*-Ebene auf dem beweglichen Träger für die optimale Positionierung in Bezug auf die Brust.

**2.** Vorrichtung gemäß Anspruch 1, **dadurch gekennzeichnet, dass** der erste Detektor (10) einen Kollimator des Lochblenden-Typs verwendet.

**3.** Vorrichtung gemäß Anspruch 1 oder 2, **dadurch gekennzeichnet, dass** der zweite Detektor (20) einen Kollimator des Typs mit parallelen Löchern verwendet.

**4.** Vorrichtung gemäß einem der Ansprüche 1 bis 3, **dadurch gekennzeichnet, dass** der erste Detektor (10) einen NaI (T1)- gepixelten Szintillator verwendet.

**5.** Vorrichtung gemäß einem der Ansprüche 1 bis 3, **dadurch gekennzeichnet, dass** der erste Detektor (10) einen LaBr$_3$ (Ce)- Szintillator verwendet.

**6.** Vorrichtung gemäß einem der Ansprüche 1 bis 5, **dadurch gekennzeichnet, dass** das Verhältnis zwischen den Pixeldimensionen der Szintillatoren für den ersten und zweiten Szintillator zwischen 1 und 1.4 mm ist.

**7.** Vorrichtung gemäß einem der Ansprüche 1 bis 6, **dadurch gekennzeichnet, dass** der zweite Detektor (20) einen LaBr$_3$ (Ce)- Szintillator verwendet.

**8.** Vorrichtung gemäß einem der Ansprüche 1 bis 6, **dadurch gekennzeichnet, dass** der zweite Detektor (20) einen NaI (T1)- gepixelten Szintillator verwendet.

**9.** Vorrichtung gemäß einem der Ansprüche 1 bis 8, **dadurch gekennzeichnet, dass** die Dimensionen des ersten Detektors (10) 45 - 55 * 45 - 55 mm$^2$ sind, um die Spot-Kompression zu erlauben.

**10.** Vorrichtung gemäß einem der Ansprüche 1 bis 9, **dadurch gekennzeichnet, dass** das Sichtfeld variiert wird, wobei Kunststoffabstandhalter mit diesem Fenster verwendet werden, um die Optimierung der Sichtfeld-Abstimmung Effektivität-Ortsauflösung für die Optimierung der Nachweisbarkeit von kleinen Läsionen zu erlauben.

**Revendications**

**1.** Dispositif pour détecter de petites tumeurs ou lésions dans le diagnostic du cancer du sein à l'aide d'une imagerie moléculaire utilisant des radionucléides, comprenant un premier et un deuxième détecteurs, entre lesquels le sein est destiné à être positionné, **caractérisé en ce que** :

- ledit deuxième détecteur (20) a des dimensions identiques à celles du film mammographique, et ledit premier détecteur (10) a des dimensions qui sont inférieures à celles du film mammographique, et telles qu'elles sont appropriées pour réaliser une compression par points, et, par conséquent, réduire la distance lésion-détecteur ;
- ledit deuxième détecteur (20) est monté de façon fixe sur un support (30) qui s'étend dans une direction z ;
- ledit premier détecteur (10) est monté :

o sur un support mobile (30) le long de ladite direction z (30), pour la compression du sein,
o d'une façon rotative sur ledit support mobile de façon à être approprié pour former un angle α entre 0 et 90 degrés avec la direction z, de façon à minimiser la distance lésion-détecteur dans les cas de tumeurs proches du thorax, et
o d'une façon mobile dans le plan xy sur ledit support mobile pour le positionnement optimal par rapport au sein.

**2.** Dispositif selon la revendication 1, **caractérisé en ce que** ledit premier détecteur (10) utilise un collimateur du type à trou d'aiguille.

**3.** Dispositif selon la revendication 1 ou 2, **caractérisé en ce que** ledit deuxième détecteur (20) utilise un collimateur du type à trous parallèles.

**4.** Dispositif selon l'une quelconque des revendications 1 à 3, **caractérisé en ce que** ledit premier détecteur (10) utilise un scintillateur pixellisé au NaI(Tl).

**5.** Dispositif selon l'une quelconque des revendications 1 à 3, **caractérisé en ce que** ledit premier détecteur (10) utilise un scintillateur au LaBr$_3$(Ce).

**6.** Dispositif selon l'une quelconque des revendications 1 à 5, **caractérisé en ce que** le rapport entre les dimensions de pixels des scintillateurs est compris entre 1 et 1,4 mm pour les premier et deuxième scintillateurs.

7. Dispositif selon l'une quelconque des revendications 1 à 6, **caractérisé en ce que** ledit deuxième détecteur (20) utilise un scintillateur au LaBr$_3$(Ce).

8. Dispositif selon l'une quelconque des revendications 1 à 6, **caractérisé en ce que** ledit deuxième détecteur (20) utilise un scintillateur pixellisé au NaI(Tl).

9. Dispositif selon l'une quelconque des revendications 1 à 8, **caractérisé en ce que** les dimensions dudit premier détecteur (10) sont de 45 à 55 x 45 à 55 mm$^2$ afin de permettre la compression par points.

10. Dispositif selon l'une quelconque des revendications 1 à 9, **caractérisé en ce que** le champ de vision est amené à varier à l'aide d'éléments d'espacement en matière plastique avec cette fenêtre, de façon à permettre l'optimisation du compromis rendement-définition spatiale du champ de vision pour l'optimisation de la faculté de détection de petites lésions.

Fig. 1

(a)    (b)    (c)

Fig. 2

EP 2 342 586 B1

Fig. 3

Fig. 4

EP 2 342 586 B1

PMT
Crystal
Collimator

Mamma
Deep lesion

FOV at the lesion height

PMT
Crystal
Collimator

Mamma
Superficial Lesion

FOV at the lesion height
Partially out-of-field lesion

PMT
Crystal
Collimator
Spacer

Mamma
Superficial Lesion

FOV at the lesion height

Fig. 5

Fig. 6

Fig. 7

EP 2 342 586 B1

Fig. 8

## REFERENCES CITED IN THE DESCRIPTION

### Patent documents cited in the description

- US 6583420 B

### Non-patent literature cited in the description

- **D.B. KOPANS.** *AJR,* 1992, vol. 158, 521
- **BIRD RE et al.** Analysis of cancers missed at screening mammography. *Radiology,* 1992, vol. 184, 613-617
- **D. CYRLAK.** *Radiology,* 1988, vol. 168, 661
- **F. SCOPINARO et al.** *NIM A,* 2003, vol. 497, 14-20
- **I. KHALKHALI et al.** *J. Am Coll. Surg.,* 1994, vol. 178, 491
- **G. DE VINCENTIS et al.** *NIM A,* 2003, vol. 497, 46-50
- **D. KIEPER.** *NIMA,* 2003, vol. 497, 168-173
- **C.B. HRUSKA et al.** *Physica Medica,* 2006, vol. 21 (1), 72
- **R. PANI et al.** *NIM A,* 1997, vol. 392, 295
- **P.G. JUDY et al.** *IEEE-NSS 2007 Conference records,* 2007, 4040-4043
- **G.J. GRUBER et al.** *IEEE TNS NS,* 1999, vol. 46, 2119-2123
- **F. GARIBALDI et al.** *NIM A,* 2006, vol. 569, 286-290
- **M.N. CINTI et al.** *IEEE TNS,* 2003, vol. 50 (5), 53-59
- **E. CISBANI et al.** *NIM A,* 2007, vol. 571, 169-172
- **F. CUSANNO et al.** *NIM A,* 2006, vol. 569, 193-196
- *Saint-Gobain Crystals, personal communication,* March 2007
- **R. PANI et al.** *NIM A,* 2006, vol. 567, 294-297
- **F. GARIBALDI et al.** *Nucl. Instr. Meth A,* 2001, vol. 471, 222-228
- **M.L. MAGLIOZZI et al.** *Proceedings of 9th ICATPP Conference,* 2005
- **O' CONNOR.** *Am. J. Roentgentology*
- **K. MADSEN.** *THE JOURNAL OF NUCLEAR MEDICINE,* April 2007, vol. 48 (4
- **M. L. MAGLIOZZI et al.** GEANT4 Code for Optimization of Molecular Imaging Detector. *ISTISAN Reports 06/14,* 15-19
- *Saint-Gobain Cristals, BrilLanCe Scintillators performance summary, Scintillation Products Technical Note,* December 2007
- **MK. O'CONNOR et al.** *Proc. Of SPIE,* 2006, vol. 6319, 6319 D-1 6319 D15